# EUROPEAN PATENT APPLICATION

(11) **EP 4 717 296 A1**
(43) Date of publication of application: **01.04.2026**
(21) Application number: 24202562.5
(22) Date of filing: 25.09.2024
(51) Int. Cl.: A61M 25/00, A61M 25/01, B29C 48/21

(54) **COEXTRUDED OUTER SHAFT DESIGN FOR ROLLING MEMBRANE CATHETERS**

(71) Applicant: BIOTRONIK AG, 8180 Bülach (CH)
(72) Inventor: BARDILL, Anita, 8700 Küsnacht (CH); SCHWITZER, Alwin, 8180 Bülach (CH)
(74) Representative: WSL Patentanwälte Partnerschaft mbB

(57) **Abstract**

A rolling membrane catheter (100) comprises a rolling membrane (120) and a coextruded outer shaft (130) comprising an inner layer (132) and an outer layer (134). A method for coextruding a coextruded outer shaft comprises coextruding an outer shaft and attaching a rolling membrane to the outer shaft.

## Description

The present invention relates to rolling membrane catheters and methods for coextruding outer shafts, e.g., of a rolling membrane catheter.

Medical treatment of a stenosis, e.g., via percutaneous coronary intervention, may involve the insertion of a catheter into a blood vessel at the position of the stenosis. Rolling membrane catheters allow catheter insertion into narrow cavities by everting a rolling membrane during a roll-out process.

Typically, in a first optional step, a guidewire is introduced into the blood vessel and guided to the treatment site, e.g., the location of a stenosis. The rolling membrane of the rolling membrane catheter may be everted/rolled out, e.g., by providing pressure to the inside of the rolling membrane and moving one end of the rolling membrane distally. This may cause an eversion of the rolling membrane along the vessel walls, for example, without substantive friction. The one end of the rolling membrane may be attached to, e.g., an inner shaft which moves distally (e.g., relative to an outer shaft of the rolling membrane catheter) when the rolling membrane, attached to a distal end of the inner shaft, is everted. This everts the rolling membrane in a distal direction towards (and possibly past) a stenosis. The rolling membrane can for example widen the stenosis or provide a guide catheter for other means to be introduced to the blood vessel at the location of the stenosis.

The roll-out and roll-in of the rolling membrane and the resulting friction between the outer shaft and the inner shaft lead to stress on the material. As a result, there is a need for robust rolling membrane catheters which a stable and easy to use at the same time.

The above need is at least partly met by the aspects as described herein.

According to a first aspect of the present invention, a rolling membrane catheter comprises a rolling membrane and a coextruded outer shaft comprising an inner layer and an outer layer.

Using a coextruded outer shaft in a rolling membrane catheter with a rolling membrane poses multiple advantageous technical effects: Firstly, having an outer and an inner layer allows optimization of the coextruded outer shaft in terms of the requirements to its interaction with its surrounding (e.g., when moving the catheter into a cavity, whereby the outer layer may be in contact, e.g., sliding contact with the surrounding and/or the outer layer may be required to provide sufficient stability on the one hand and sufficient flexibility on the other hand to adapt the shape of the catheter to the shape of the respective cavity). Additionally or alternatively, the outer layer may be optimized for bonding to the rolling membrane. At the same time, the inner layer may be optimized in terms of the requirements to its interaction with an inner member of the rolling membrane catheter, e.g., an inner shaft.

Using such coextruded outer shaft in a rolling membrane catheter with a rolling membrane provides a non-intuitive solution. Surprisingly, the inventors found that providing an outer shaft in a rolling membrane catheter wherein typically the rolling membrane is placed at least partly between an inner shaft and the outer shaft does not exacerbate potential space problems due to the limited cavity size but rather results in reduced friction between the members of the rolling membrane catheter, improves mechanical properties (e.g., stability and/or flexibility of the rolling membrane catheter) and thus reduces forces during a typical use of the rolling membrane catheter (e.g., rolling membrane eversion, and retraction). In comparison to providing a coextruded inner shaft, the outer shaft (as it interacts with the surrounding of the rolling membrane catheter) may play a more decisive role in terms of the mechanical properties which allows to optimize these accordingly.

In some examples, the inner layer may be adapted to reduce friction with an inner shaft to be used with the rolling membrane catheter.

This may particularly advantageously allow for a friction-reduced relative motion of the outer and inner shaft of the rolling membrane catheter which may be particularly advantageous as, despite the critical size restrictions, this optimizes the roll-in and roll-out process typically performed by such rolling membrane catheter. This allows to accelerate their handling which may be decisive for treatment success and increase reliability, prevent malfunction and thus damage to the rolling membrane catheter, e.g., damage to the membrane and/or dispatching from the outer and/or inner shaft.

In some examples, the inner layer may comprise a polyethylene (PE), preferably a high-density polyethylene, HDPE, and a cyclic olefin copolymer, COC, preferably an amorphous COC.

These materials may be particularly suitable for the following purposes:
PE may typically be semi-crystalline and has many advantageous properties and can be customized to the respective application by means of the selected manufacturing process and the addition of additives. As a thermoplastic that softens at temperatures above +80°C, it is easy to process, particularly in (co-)extrusion. PE is resistant to most acids and alkalis, but also to oils, greases, petrol and aliphatic hydrocarbons which is critical for insertion in cavities that may comprise such liquids. HDPE comprises only a few branched polymer chains and therefore a higher density of ca. 0.94 to 0.97 g/cm³ which may lead to a further improvement in the mentioned regards.

The properties of COCs can be modified over a wide range by changing the incorporation ratios of cyclic and linear olefins. Essentially, the heat deflection temperature is adjusted in a range from 65 to 175°C. This may be particularly advantageous as this allows controlling the coextrusion advantageously and/or simplify it.

PE, e.g., HDPE, may typically be semi-crystalline. In contrast, cyclic olefin copolymer (COC) may be amorphous. When the mixture comprises (e.g., granules of) both polymers, it serves as a preliminary product. Through appropriate melting and mixing methods, these (granules) can form a copolymer with significantly lower friction forces compared to semi-crystalline PE (e.g., HDPE), especially against metallic surfaces. Experimental comparisons showed an approximately 30% reduction in friction forces.

In some examples, the inner layer may comprise a mixture comprising or consisting of PE and COC, wherein ≥ 70% by weight, preferably ≥ 90% by weight, and still more preferably 100% by weight, relative to the total mass of PE, of PE in the mixture is HDPE.

In some examples, the mixture of PE and COC comprising a weight percentage relationship between the total mass of PE and the total mass of COC in the mixture of 60 to 98% by weight to 40 to 2% by weight, relative to the total mass of PE and COC in the mixture.

Alternatively, in some examples the inner layer may comprise a mixture of PE and COC, wherein preferably 70% or more, 80% or more, or 90% or more of the mixture may be PE, preferably HDPE.

Optimal friction reduction, particularly with metallic friction partners, is achieved when the weight ratios of PE to COC in the mixture are in the above ranges.

In some examples, wherein the outer layer comprises at least one of: a polyamide, PA, preferably PA 12, a polyether block amide, a polyester, and a polyester elastomer.

Polyamides, PAs, for example, have good chemical resistance to, e.g., organic solvents, are easy to process, have good strength, rigidity, and toughness, and/or yield high wear resistance and good sliding properties. The same applies analogously to polyether block amides, polyesters, and polyester elastomers.

Further, the rolling membrane may, e.g., comprise PA, preferably PA 12, a polyether block amide, a polyester, and a polyester elastomer. The match between the outer layer and rolling membrane material facilitates attaching the rolling membrane to the outer shaft which may increase stability and reliability.

In some examples, the coextruded outer shaft may further comprise an intermediate layer between the outer layer and the inner layer.

The intermediate layer may, e.g., be configured to promote adhesion between the inner layer and the outer layer as described herein.

In some examples, the intermediate layer may be configured to mechanically couple the inner layer and the outer layer and/or the intermediate layer may comprise a low-density polyethylene, LDPE, preferably a maleic acid anhydride-grafted LDPE.

E.g., when using maleic acid anhydride-grafted LDPE as an intermediate layer, it could be shown by the inventors that physical bonds to the inner layer (e.g., comprising a mixture of PE and COC) and chemical bonds to the outer layer (e.g., comprising PA) may be established to promote adhesion between the inner layer and the outer layer.

In some examples, the coextruded outer shaft may comprise three layers and/or may be free of a coating.

Having only three layers may provide a simple yet optimized rolling membrane catheter/outer shaft.

Generally, there are multiple disadvantages associated with coatings: Adding a coating to a catheter surface, for example, with a hydrophobic and/or hydrophilic coating agent, is associated with the risk that these coatings, particularly hydrophilic coatings may interact with biological liquids (e.g., blood) and may release substances, which es preferably avoided. Therefore, it becomes apparent why avoiding a coating is technically advantageous. Further, this saves costs and material.

Being free of coating may equally be referred to as a scenario, wherein the outer layer is the outermost layer of the (coextruded) outer shaft and the inner layer is the innermost layer of the (coextruded) outer shaft.

In some examples, at least one layer, preferably each layer, of the coextruded outer shaft may comprise essentially the same cross section at at least two axial positions along the coextruded outer shaft, preferably along essentially the whole coextruded outer shaft in an axial direction.

This may, e.g., be the case for a (hollow) cylindrical outer shaft which is the preferred shape for the outer shaft for safe and reliable use of the rolling membrane catheter. Avoiding surface unevenness may increase safety.

In some examples, the coextruded outer shaft may comprise an outer diameter of 1.00 mm to 1.50 mm, preferably of 1.30 mm to 1.40 mm and/or the coextruded outer shaft may comprise an inner diameter of 0.8 mm to 1.3 mm, preferably of 1.00 mm to 1.1 mm.

In various tests, these diameter ranges proved advantageous: These tests comprise testing contrast media passage/flow through a side port of the introducer sheath while the rolling membrane catheter was engaged in the introducer sheath and attaching an X-ray marker band with a diameter of ca. 1.3 mm to 1.5 mm to the rolling membrane catheter, e.g., the outer shaft and/or the inner shaft, preferably a distal portion thereof. Even with such X-ray marker band suitably sized rolling membrane catheters (e.g., as per the above ranges) could be smoothly inserted into a test cavity. At the same time, it could be ensured that a maximum diameter of 1.52 mm was not exceeded. The rolling membrane catheter may, e.g., comprise the X-ray marker band, e.g., attached via gluing, embedding, and/or rotary swaging.

In some examples, the rolling membrane may be attached, preferably by welding, to the outer shaft, preferably to the outer layer, more preferably to a distal portion of the outer layer.

Thereby, the rolling membrane may be in place to allow the inner shaft to move distally relative to the outer shaft upon membrane eversion. This may, e.g., improve safety with a compact catheter design at the same time.

In some examples, the rolling membrane catheter may further comprise an inner shaft configured to be placed at least partly radially inside the outer shaft and to be moved relative to the outer shaft in an axially proximal and/or distal direction.

The inner shaft may, e.g., be configured to be moved distally (e.g., into a cavity) relative to the outer shaft as described herein: E.g., the rolling membrane of the rolling membrane catheter may be everted, e.g., by providing pressure to the inside of the rolling membrane and moving one end of the rolling membrane distally causing the eversion of the rolling membrane. One end of the rolling membrane may be attached to, e.g., the inner shaft which may move distally (e.g., relative to an outer shaft of the rolling membrane catheter) when the rolling membrane, e.g., attached to a distal end of the inner shaft, is everted.

In some examples, the rolling membrane may be attached, preferably by welding, to the inner shaft, preferably to a distal portion of the inner shaft.

In some examples, the rolling membrane catheter may further comprise a fluid unit for determining a fluid pressure and/or a fluid flow of the rolling membrane; and/or a hub for inserting one or more devices into the rolling membrane catheter.

Determining the fluid state of any member may provide a useful control over various devices which is compatible with the required flexibility of the used catheters and/or devices.

The fluid state may relate for example to a fluid pressure and/or a fluid flow. The fluid may be any liquid fluid, gaseous fluid, and/or any mixture thereof. For example, the fluid unit may be a pressure pump for providing a fluid pressure and/or a fluid flow and/or a fluid sensor for sensing any parameter associated with the fluid state.

The at least one fluid unit may contribute to and/or induce different modes in which, e.g., the rolling membrane catheter may be used. For example, a constant fluid pressure and/or constant fluid flow may be provided to the rolling membrane during eversion and/or retraction of the rolling membrane to continuously keep the rolling membrane in an at least partly inflated state facilitating the rolling motion of the rolling membrane. In some examples closed-loop configurations may be implemented in which a fluid state is induced and simultaneously sensed.

According to a second aspect of the present invention, a method for coextruding a coextruded outer shaft comprises coextruding an outer shaft and attaching a rolling membrane to the outer shaft.

Thereby, a coextruded outer shaft with the advantages described herein may be produced in an efficient fashion that saves energy, costs, and resources.

Coextrusion of a coextruded outer shaft (e.g., comprising an inner layer, an intermediate layer, and an outer layer) may, e.g., be performed as follows. Notably, the overall methodological concept described below may analogously be applied to an outer shaft with less, more and/or different layers:
In a first step, the precursors, in this example three components may be melted: For the outer layer, e.g., the polyamide-based outer layer, a first component, e.g., PA 12 (e.g., Grilamid L25 from EMS-CHEMIE) may be melted in a first extruder. For the (e.g., adhesion-promoting) intermediate layer, a second component, e.g., a maleic acid anhydride-grafted LDPE (e.g., Admer^{®} NF 408-E, from Mitsui) may be melted in a second extruder. For the inner layer, a third component, e.g., a mixture consisting of 90% wt/wt granules of a semi-crystalline HDPE (e.g., Bormed^{®} HE2581-PH from Borealis) and 10% wt/wt of the (e.g., amorphous) COC (e.g., TOPAS^{®} 6013 from TOPAS) may be melted in a third extruder.

The separate (prepared) melts may be delivered to a tube shaping means and the individual melts may be arranged such that the melt of the first extruder forms the outer layer, the melt of the second extruder forms the (e.g., adhesion-promoting) intermediate layer, and the melt of the third extruder forms the inner layer of the coextruded outer shaft. The inner layer thus created forms a lumen of the coextruded tube.

In some exemplary coextrusions, the melting temperatures may be adapted to the respective layer. For the above exemplary layer materials, the following temperatures may be used: For the first extruder, rising temperatures from 230°C to 250°C may be used, for the second extruder rising temperatures from 210°C to 225°C may be used, and for the third extruder rising temperatures from 210°C to 225°C may be used.

It is noted that all functions described herein may be implemented as a corresponding functionality (means) of the rolling membrane catheter described herein and/or as a corresponding step of the methods outlined herein. Even if described with reference to the rolling membrane catheter (and/or one or more or its means/components), the features outlined herein may be realized analogously in a respective step of a method as described herein.
- Fig. 1A: shows a schematic representation of a rolling membrane catheter with a rolling membrane and a guidewire in a partly everted state.
- Fig. 1B: shows a schematic representation of a rolling membrane catheter with a rolling membrane and a guidewire in a further everted state.
- Fig. 2: shows an exemplary schematic representation of a cross section of a coextruded outer shaft.
- Fig. 3A: shows an exemplary rolling membrane catheter with a two-part outer shaft.
- Fig. 3B: shows an exemplary rolling membrane catheter with a single-part outer shaft.
- Fig. 4: shows exemplary test measurements for a plurality of rolling membrane catheters.
- Fig. 5: shows a comparison of measured force peaks during retraction for a plurality of rolling membrane catheters as a function of the outer shaft diameter and of the coating.

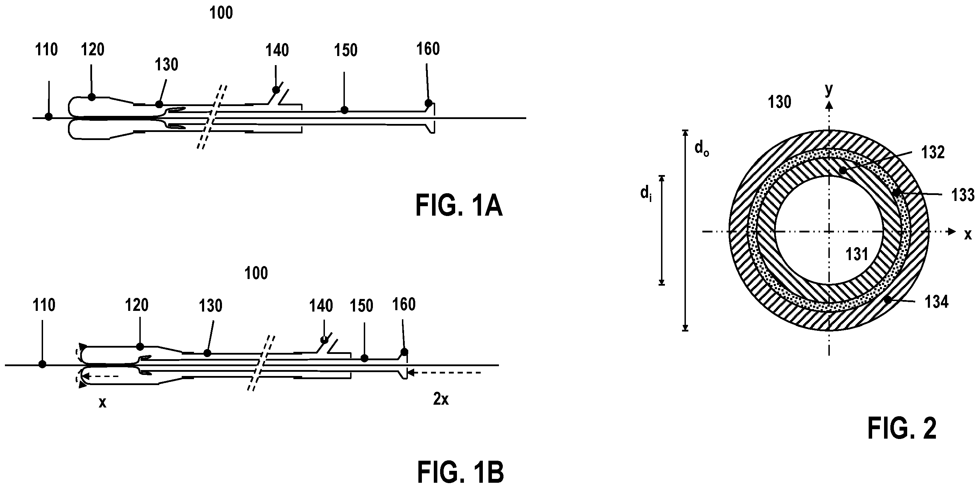

Fig. 1A shows a schematic representation of a rolling membrane catheter 100 with a rolling membrane 120 and a guidewire 110 in a partly everted state. Fig. 1B shows a schematic representation of the rolling membrane catheter 100 of Fig. 1A in a further everted state. In Figs. 1A and 1B, a distal direction, whenever referred to, relates to a leftward direction and a proximal direction, whenever referred to, relates to a rightward direction.

The catheter comprises a (coextruded) outer shaft 130 and inner shaft 150. A fluid inlet 140 is provided and configured to be connected to a fluid unit determining the fluid state of the rolling membrane 120 by providing, e.g., a fluid pressure to the rolling membrane volume limited by the rolling membrane 120, the outer shaft 130, and the inner shaft 150. In the example of Figs. 1A and 1B, the rolling membrane 120 is attached on the one hand, e.g., by welding, to a distal portion of the outer shaft 150, e.g., to the outer layer, and on the other hand, e.g., by welding, to a distal portion of the inner shaft 130.

The inner shaft 150 is in the example of Figs. 1A and 1B placed at least partly radially inside the outer shaft 130. It may, e.g., be moved relative to the outer shaft 130 in an axially proximal and/or distal direction. The exemplary embodiment of Figs. 1A and 1B further comprises a handle 160 at the proximal end of the inner shaft 150. A health professional may move the inner shaft 150 relative to the outer shaft 130 by this handle 160. They may move the inner shaft 150 in the distal direction to evert the rolling membrane 120 and in the proximal direction to retract the rolling membrane 120. As it can be seen from the comparison of Figs. 1A and 1B, upon eversion, the inner shaft 150 moves distally (leftward in Figs. 1A and 1B) to allow for an eversion of the rolling membrane 120 attached to the inner shaft 150. During both, eversion and retraction, the rolling membrane 120 is, e.g., in an inflated state such that it is everted and/or retracted in a rolling motion.

Fig. 1B shows a schematic representation of the rolling membrane catheter 100 of Fig. 1A in a further everted state. The inner shaft 150 is in a different position, dislocated by a distance x compared to the situation of Fig. 1A. The rolling membrane 120, which, in the example of Figs 1A and 1B is attached to the inner shaft 150 and in its motion coupled thereto, is also dislocated by a distance 2x in the distal direction compared to its position shown in Fig. 1A. Fig. 1B thus shows an exemplary scenario for coupled motion of the inner shaft 150 and the rolling membrane 120 bearing the risk of unwanted damage of the rolling membrane 120 when the motion of the inner shaft 150 relative to the outer shaft (in the example of Figs. 1A and 1B the two means the rolling membrane 120 is attached to) is restricted, e.g., by friction between the inner shaft 150 and the outer shaft 130. Together, Fig. 1A and 1B illustrate the basic working principle and the basic building blocks of an exemplary rolling membrane catheter 100.

Fig. 2 shows an exemplary schematic representation of a cross section of a coextruded outer shaft 130. The cross section in Fig. 2 is shown in the x-y-plane which is essentially orthogonal to the axial direction of the outer shaft 130. In the example of Fig. 2, the coextruded outer shaft 130 comprises three layers 132, 133, 134 around a cavity 131 inside of the coextruded outer shaft 130. In detail, the exemplary coextruded outer shaft 130 of Fig. 2 comprises an inner layer 132, an intermediate layer 133, and an outer layer 134, wherein the intermediate layer 133 lies radially between the inner layer 132 and the outer layer 134. The cavity 131 may, e.g., be sized and/or adapted to receive an inner shaft (not shown) as described herein. The layers 132, 133, 134 may have an (e.g., circumferentially and/or axially) essentially constant radial thickness. The radial thicknesses of the layers 132, 133, 134 may be adapted to the specific needs for a certain coextruded outer shaft 130 and in other relative proportions than shown in Fig. 2.

The exemplary coextruded outer shaft 130 of Fig. 2 comprises an outer diameter dₒ of, e.g., 1.00 mm to 1.50 mm, preferably of 1.30 mm to 1.40 mm. The exemplary coextruded outer shaft 130 of Fig. 2 comprises an inner diameter dᵢ (corresponding to the diameter of the cavity 131) of, e.g., 0.8 mm to 1.3 mm, preferably of 1.00 mm to 1.1 mm. The catheter may have a circular cross section, e.g., as shown in Fig. 2, and/or other cross sections (e.g., of an elliptical shape, hexagonal shape, regular (or irregular) polygon-shape and/or other shape).

For example, the surfaces of the coextruded outer shaft 130 may be defined by the outer surface of the outer layer 134 and the inner surface of the inner layer 132, both of which may be free of any further coating that may be rendered obsolete by such coextruded outer shaft 130.

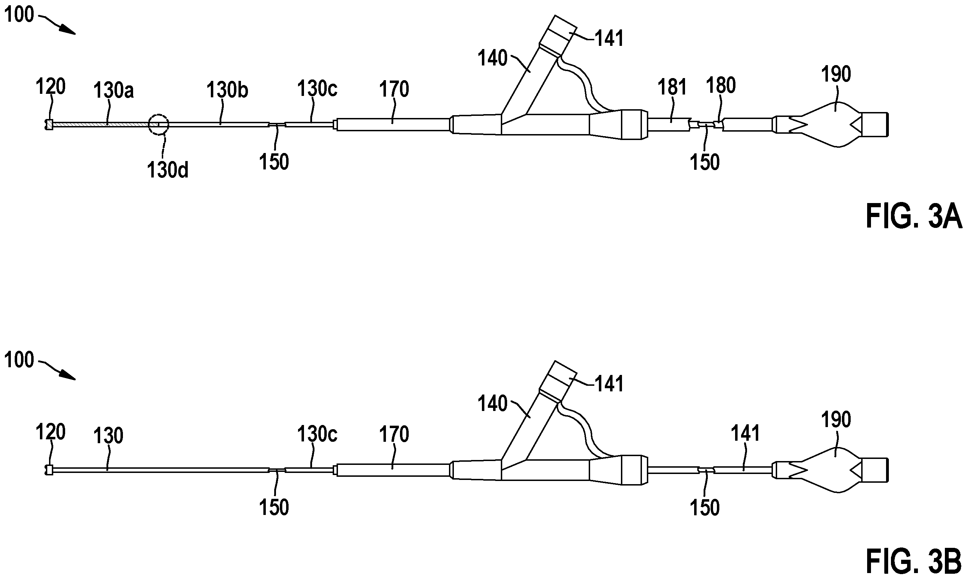

Fig. 3A shows an exemplary rolling membrane catheter 100 with a two-part outer shaft 130.

Fig. 3B shows an exemplary rolling membrane catheter 100 with a single-part outer shaft 130.

Both catheters 100 of Figs. 3A and 3B may comprise a rolling membrane 120, an inner shaft 150, a connector 140, preferably a T-connector, which may e.g., like in Figs. 1A and 1B comprise a fluid unit, with a means 141 configured to connect further equipment, e.g., a Luer taper (a standardized assembly of miniature fluid couplings designed to establish secure, leak-proof connections between a male-tapered fitting and a corresponding female component), a kink protector 170, a metal shaft 180, a safety tab 181, and/or a hub 190.

In the example of Fig. 3A, the two-part outer shaft 130 comprises a distal portion 130a and a proximal portion 130b (e.g., a proximal portion 130b with a coating, preferably a hydrophobic coating on its outer surface), wherein the proximal portion 130b may comprise a coextruded outer shaft as described herein and/or the proximal portion 130b may, e.g., comprise polyether ether ketone (PEEK) and/or the distal portion 130a may comprise a coextruded shaft as described herein. The distal portion 130a and the proximal portion 130b may, e.g., be connected by a proximal outer shaft/distal outer shaft connection.

In the example of Fig. 3B, the outer shaft 130 may comprise a coextruded outer shaft as described herein, e.g., with a coating, preferably a hydrophobic coating, on its outer surface.

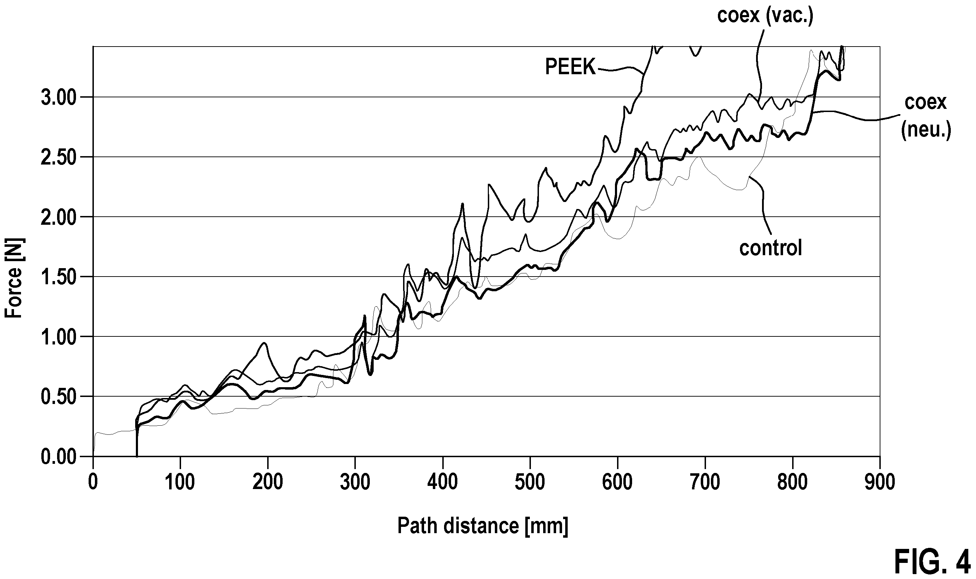

Fig. 4 shows exemplary test measurements for a plurality of rolling membrane catheters (the test is a trackability test which measures the applied force to move a catheter), namely a control catheter ("control", which may be a balloon catheter), a catheter with a PEEK outer shaft ("PEEK"), and two catheters with a coextruded outer shaft, one at vacuum conditions ("coex. vac.") and one under neutral pressure (water) ("coex. neu."). In the tests, the force on the catheter is measured as a function of the path distance of the catheter through a model system cavity. It can be seen that the catheters with the coextruded outer shafts perform equally well (i.e., low forces at essentially the same path distance) as the control catheter (but with the advantages described herein with respect to rolling membrane catheters, e.g., reduced friction between the inner and the outer shaft and less risk of cavity damage and/or catheter damage) and outperform the PEEK-based catheter which yields higher forces. This underlines that the coextruded outer shafts as per the present invention yield advantageous mechanical properties (particularly regarding stability, flexibility, and/or friction with the cavity wall).

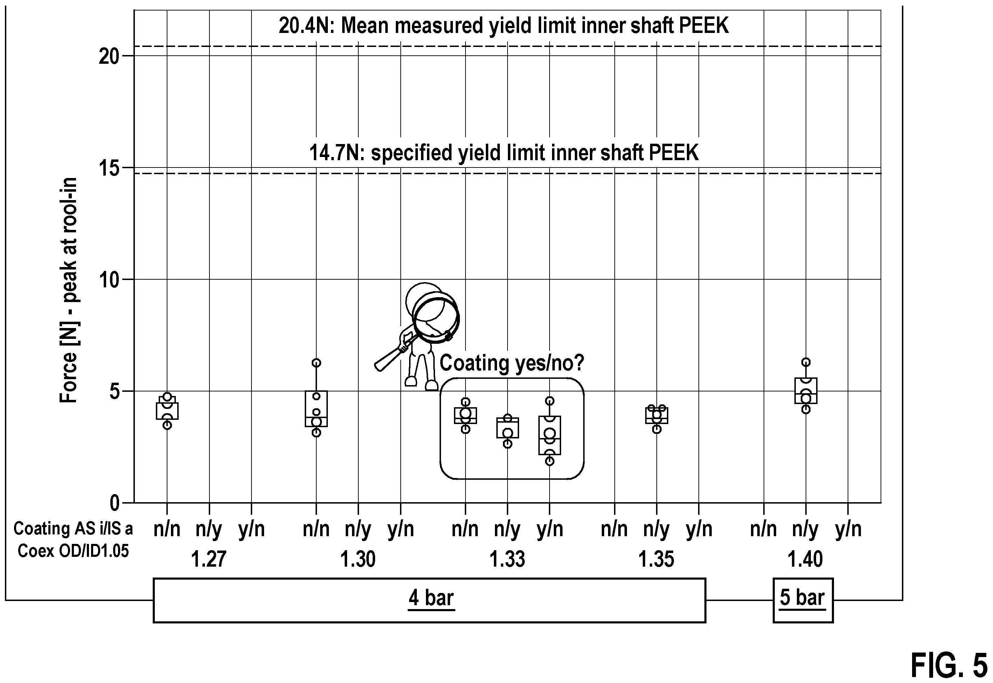

Fig. 5 shows a comparison of measured force peaks during roll-out for a plurality of rolling membrane catheters as a function of the outer shaft diameter and of the coating.

In detail, rolling membrane catheters with an outer diameter of 1.27 mm, 1.30 mm, 1.33 mm, 1.35 mm, and 1.40 mm were tested by the inventors. The 1.27 mm and 1.30 mm catheters had no coating on the outer shaft or the inner shaft. The 1.35 mm and 1.40 mm catheters had no coating on the outer shaft but a coating on the inner shaft. Three 1.3 mm catheters were tested: one with no coating on the outer shaft or the inner shaft ("n/n"), one with no coating on the outer shaft but a coating on the inner shaft ("n/y"), one with no coating on the inner shaft but a coating on the outer shaft ("y/n"). As it can be seen for all parameters, the force peak is in the range below ca. 6 N for all tested embodiments.

Further, the force peaks are well below the mean measured yield limit (inner shaft PEEK) of 20.4 N and the specified yield limit (inner shaft PEEK) of 14.7 N.

Importantly, the inventors could show that the mechanical influence of the coating is essentially negligible such that the coating may be removed in order to avoid medical risks associated therewith: For example, coatings may dissolve and contaminate the treated cavity which may yield health risks for a patient. It is advantageous to avoid these. The present invention allows to do this without suffering mechanically disadvantageous effects.

## Claims

1. Rolling membrane catheter (100) comprising:
a rolling membrane (120); and
a coextruded outer shaft (130) comprising an inner layer (132) and an outer layer (134).

2. The rolling membrane catheter (100) of claim 1, wherein the inner layer (132) is adapted to reduce friction with an inner shaft (150) to be used with the rolling membrane catheter (100).

3. The rolling membrane catheter (100) of claim 1 or 2, wherein the inner layer (132) comprises a polyethylene (PE), preferably a high-density polyethylene, HDPE, and a cyclic olefin copolymer, COC, preferably an amorphous COC.

4. The rolling membrane catheter (100) of claim 3, wherein the inner layer (132) comprises a mixture comprising or consisting of PE and COC, wherein ≥ 70% by weight, preferably ≥ 90% by weight, and still more preferably 100% by weight, relative to the total mass of PE, of PE in the mixture is HDPE.

5. The rolling membrane catheter (100) of any of claims 1 to 4, wherein the outer layer (134) comprises at least one of: a polyamide, preferably polyamide 12, a polyether block amide, a polyester, and a polyester elastomer.

6. The rolling membrane catheter (100) of any of claims 1 to 5, wherein the coextruded outer shaft (130) further comprises an intermediate layer (133) between the outer layer (134) and the inner layer (132).

7. The rolling membrane catheter (100) of claim 6, wherein the intermediate layer (133) is configured to mechanically couple the inner layer (132) and the outer layer (134); and/or
wherein the intermediate layer (133) comprises a low-density polyethylene, LDPE, preferably a maleic acid anhydride-grafted LDPE.

8. The rolling membrane catheter (100) of any of claims 1 to 7, wherein the coextruded outer shaft (130) comprises three layers and/or is free of a coating.

9. The rolling membrane catheter (100) of any of claims 1 to 8, wherein at least one layer, preferably each layer, of the coextruded outer shaft (130) comprises essentially the same cross section at at least two axial positions along the coextruded outer shaft (130), preferably along essentially the whole coextruded outer shaft (130) in an axial direction.

10. The rolling membrane catheter (100) of any of claims 1 to 9, wherein the coextruded outer shaft (130) comprises an outer (dₒ) diameter of 1.00 mm to 1.50 mm, preferably of 1.30 mm to 1.40 mm; and/or wherein the coextruded outer shaft (130) comprises an inner (dᵢ) diameter of 0.8 mm to 1.3 mm, preferably of 1.00 mm to 1.1 mm.

11. The rolling membrane catheter (100) of any of claims 1 to 10, wherein the rolling membrane (120) is attached, preferably by welding, to the outer shaft (130), preferably to the outer layer (134), more preferably to a distal portion of the outer layer (134).

12. The rolling membrane catheter (100) of any of claims 1 to 11, further comprising an inner shaft (150) configured to be placed at least partly radially inside the outer shaft (130) and to be moved relative to the outer shaft (130) in an axially proximal and/or distal direction.

13. The rolling membrane catheter (100) of claim 12, wherein the rolling membrane (120) is attached, preferably by welding, to the inner shaft (150), preferably to a distal portion of the inner shaft (150).

14. The rolling membrane catheter (100) of any of claims 1 to 13, further comprising a fluid unit for determining a fluid pressure and/or a fluid flow of the rolling membrane (120); and/or a hub for inserting one or more devices into the rolling membrane catheter (100).

15. Method for coextruding a coextruded outer shaft (130), the method comprising:
coextruding an outer shaft (130); and
attaching a rolling membrane (120) to the outer shaft (130).
